# EUROPEAN PATENT APPLICATION

(11) **EP 1 952 814 A1**
(43) Date of publication of application: **06.08.2008**
(21) Application number: 06822960.8
(22) Date of filing: 27.10.2006
(51) Int. Cl.: A61K 31/7032, A61P 1/02, A61P 1/04, A61P 3/10, A61P 5/14, A61P 5/50, A61P 7/00, A61P 7/06, A61P 11/02, A61P 11/06, A61P 17/00, A61P 17/04, A61P 19/02, A61P 19/04, A61P 19/06, A61P 19/08, A61P 21/04, A61P 25/00, A61P 29/00, A61P 37/02

(54) **THERAPEUTIC AGENT COMPRISING GLYCOLIPID DERIVATIVE CAPABLE OF INHIBITING THE FUNCTION OF NKT CELL AS ACTIVE INGREDIENT**

(30) Priority: 28.10.2005 JP 2005314783
(71) Applicant: Japan as represented by President of National Center of Neurology and Psychiatry, Kodaira-shi, Tokyo 187-8551 (JP); Asubio Pharma Co., Ltd., Minato-ku Tokyo 107-8541 (JP)
(72) Inventor: MIYAKE, Sachiko, Tokyo 164-0001 (JP); YAMAMURA, Takashi, Tokyo 167-0051 (JP); ANNOURA, Hirokazu, Kyoto 617-0821 (JP)
(74) Representative: Stoner, Gerard Patrick
(86) International application number: PCT/JP2006/322042
(87) International publication number: WO 2007/049819

(57) **Abstract**

A therapeutic drug for diseases, in which NKT cells or stimulus of NKT cells is involved in deterioration of disease conditions, containing, as an active ingredient, a glycolipid derivative having the formula (I): 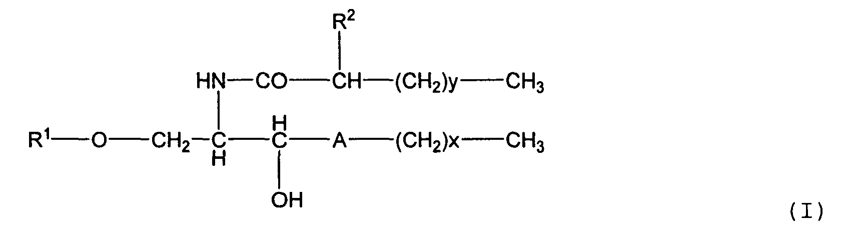 wherein R¹ indicates an aldopyranose residue, R² indicates a hydrogen atom or a hydroxy group, A indicates -CH₂-, -CH(OH)-CH₂- or -CH=CHCH₂-, x indicates an integer of 13 to 16 and y indicates an integer of 0 to 25
or its pharmacologically acceptable hydrate or solvate.

## Description

### TECHNICAL FIELD

The present invention relates to a novel glycolipid derivative useful for chronic inflammatory diseases such as multiple sclerosis, myasthenia gravis, chronic rheumatoid arthritis, juvenile rheumatoid arthritis, primary gout, systemic lupus erythematosus, Sjogren syndrome, systemic scleroderma, mixed connective tissue disease, insulin-dependent diabetes, idiopathic thrombocytopenic purpura, Hashimoto's thyroiditis, Basedow's disease, pernicious anemia, Addison's disease, atrophic gastritis, hemolytic anemia, ulcerative colitis, Crohn's disease, autoimmune hepatitus, pemphigus, pemphigoid, vasculitis syndrome, autoimmune hemolytic anemia, Goodpasture's syndrome, Behçet's disease, sarcoidosis, organ transplant rejection, and allergic diseases such as inflammatory diseases and bronchial asthma, atopic asthma, atopic dermatitis, allergic rhinitis, urticarial eruption, hayfever, drug allergy, contact dermatitis, and other allergic diseases and organ transplant rejection and the pharmacologically acceptable hydrate and solvate thereof and a drug containing the same.

### BACKGROUND ART

An immune system inherently distinguishes self and non-self and maintains the homeostasis of the body by excluding non-self components by an immune-response and by inducing immunological unresponsiveness against self components. Autoimmune diseases are considered as a condition where the breakdown of the immunological unresponsiveness against self antigens leads to the immunological attack to self components, while allergy is considered as a condition where the immune-response against non-self antigens (i.e., foreign antigens) induces problems in the body due to the excessive response.

The conventional treatment method for chronic inflammatory diseases including autoimmune diseases and allergic diseases focused mainly on non-specific immunosuppressive therapy involving glucocorticoids and immunosuppressives. Since these methods of treatment have many side effects, development of autoantigen-specific immunosuppressives has been strongly desired. Autoantigen peptide treatments were recently tested with this goal in mind. Since peptides are presented in major histocompatibility gene complexes (MHC) rich in polymorphism, there are remarkable individual differences in efficacy. While there have been improved cases, there have also been greatly deteriorated cases. These results clearly show the difficulty in the clinical application of the peptide treatment.

NKT cells are lymphocytes which express both NK cell marker (i.e., NKT receptors) and T-cell antigen receptor (TCR). While T-cells recognize peptides bound to major histocompatibility antigen (MHC), NKT-cells recognize glycolipids presented by non-polymorphic CD1d molecules as antigens. NKT cells produce a large amount of cytokines in an extremely short period of time, when stimulated through TCR.

For example, the α-galactosyl ceramide (α-GC) having the formula (II): 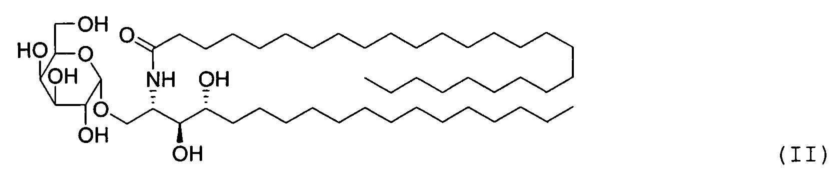 has been reported as the first glycolipid ligand to selectively activate CD1d restricted NKT cells. α-GC has been shown to exhibit an anti-tumor activity and immune-stimulatory activities (see T. Kawano et al., Proc. Natl. Acad. Sci. USA. 1998, 95, 5690. and Japanese Patent No. 3088461). Furthermore, the present inventors reported that the OCH having the formula (III) shortened in the length of the carbon chain of the sphingosine base of α-GC: 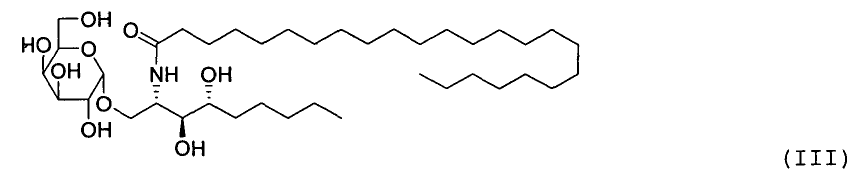 promotes the production of Th2 type cytokine and biases the Th1/Th2 immune balance toward Th2 and therefore exhibits high effectiveness in the suppression of diseases in the animal model of multiple sclerosis: murine experimental autoimmune encephalomyelitis (EAE) and the animal model of rheumatoid arthritis: collagen induced arthritis (CIA) (see K. Miyamoto et al., Nature 2001, 413, 531., A. Chiba et al., Arthritis Rheum. 2004, 50, 305., T. Yamamura et al., Curr. Top. Med. Chem. 2004, 4, 561., and WO2003/016326). That is, the expression of the action of the OCH having the formula (III) in the above autoimmune disease animal can be explained based on the "active suppression" by the production of Th2 type inhibitory cytokines from the NKT cells in charge of immune response.

On the other hand, it has been reported that NKT cells act as effecter cells involved in the deterioration of disease conditions in autoimmune disease models such as arthritis and bronchial asthma models (see O. Akbari et al., Curr. Opin. Immunol. 2003, 15, 627). Therefore, in such a disease condition, if a drug treatment suppressing the function of NKT cells could be established, this would lead to not only the prevention and treatment of autoimmune diseases, but also the treatment of allergic diseases. However, no effective drug has yet been known to suppress the function of NKT cells.

### DISCLOSURE OF THE INVENTION

In view of the above circumstances, the objective of the present invention is to provide a therapeutic drug which suppresses the function of NKT cells to thereby be effective against chronic inflammatory diseases, allergic diseases, and other diseases, in which it is known that NKT cells are involved in the deterioration of the disease conditions.

Another objective of the present invention is to provide a novel glycolipid derivative (I), acting as a ligand of CD1d restricted NKT cells, but not substantially inducing cytokine production such as IL-4, IFN-γ or other cytokine from the NKT cells and being useful as the above pharmaceuticals and their pharmacologically acceptable hydrate and solvate.

The present inventors have synthesized glycolipids which can control the autoimmune response and have been developing a therapeutic drug for the treatment of autoimmune diseases (WO2003/016326;WO2004/072091; Karl O. A. Yu et al., Proc. Natl. Acad. Sci. USA, 2005, 102, 3383). Among these, the present inventors discovered that, in a derivative having a longer length of the carbon chain of the sphingosine base of the glycolipid than α-GC (II), a surprisingly strong suppressive effect of the antibody-induced arthritis is expressed by a glycolipid derivative (hereinafter referred to as an "SGL (suppressor glycolipid)") having the formula (I): 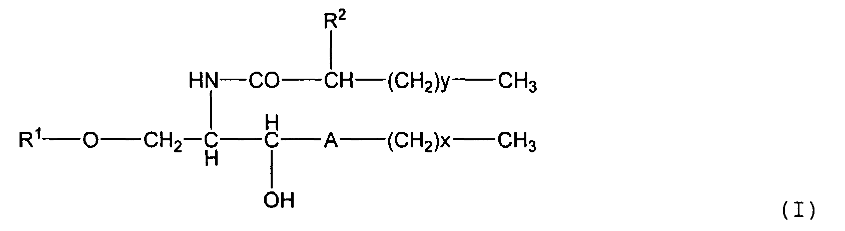 wherein R¹ indicates an aldopyranose residue, R² indicates a hydrogen atom or a hydroxyl group, A indicates -CH₂-, - CH(OH)-CH₂- or -CH=CHCH₂-, x indicates an integer of 13 to 16 and y indicates an integer of 0 to 25.

The SGL having the formula (I) induces slight proliferation of NKT cells and slight production of IFN-γ or other cytokines but remarkably suppresses the response to restimulation of NKT cells after the pretreatment of the SGL (i.e. immunological unresponsiveness). An antibody arthritis model is reported to develop the onset of arthritis in mice without NKT cells (J. Exp. Med. 2005, 201, 41-7; Arthritis Rheum. 2005, 52, 1941), but the glycolipids described in the present invention strongly suppress the onset of the arthritis. Further, the SGL having the formula (I) suppresses cellular infiltration mainly comprised of eosinophiles to the airway in the bronchial asthma animal model, suppresses the production of IL-5, IL-13 and other cytokines in alveolus washings, and suppresses airway sensitivity. That is, the present inventors discovered that the SGL having the formula (I) is an effective glycolipid derivative capable of suppressing the inflammatory response induced by autoantibodies and can form a therapeutic drug ingredient for autoimmune diseases such as autoimmune arthritis and allergic diseases such as bronchial asthma and therefore achieves the objective of the present invention.

### BRIEF DESCRIPTION OF THE DRAWINGS

The present invention will now be explained with reference to the drawings, wherein:
FIG. 1 is a graph showing the suppressive effects (clinical score) of synthesized glycolipid derivatives on K/BxN serum transferred arthritis.
FIG. 2 is a graph showing the effect (clinical score) of synthesized glycolipid derivatives on K/BxN serum transferred arthritis induced in Jα18 gene-deficient mice not having any NKT cells.
FIG. 3 is a graph showing the suppressive effects (clinical score) of synthesized glycolipid derivatives on collagen induced arthritis.
FIG. 4 is a graph showing the effects of the present compounds on NKT cells.
FIG. 5 is a graph showing the effects on NKT cells of preadministration of the present compound.
FIG. 6 is a graph showing the suppressive effects of cellular infiltration in alveolus washings in a bronchial asthma model of the present compound.
FIG. 7 is a graph showing the suppressive effects of cytokine in alveolus washings in a bronchial asthma model of the present compound.
FIG. 8 is a graph showing the suppressive effects of the present compound on lung pathological findings in a bronchial asthma model.
FIG. 9 is a graph showing the suppressive effects of cytokine in alveolus washings in a bronchial asthma model of the present compound.

### BEST MODE FOR CARRYING OUT THE INVENTION

In the present invention, as chronic inflammation diseases, for example, multiple sclerosis, myasthenia gravis, chronic rheumatoid arthritis, juvenile rheumatoid arthritis, primary gout, systemic lupus erythematosus, Sjogren syndrome, systemic scleroderma, mixed connective tissue disease, insulin-dependent diabetes, idiopathic thrombocytopenic purpura, Hashimoto's thyroiditis, Basedow's disease, pernicious anemia, Addison's disease, atrophic gastritis, hemolytic anemia, ulcerative colitis, Crohn's disease, autoimmune hepatitus, pemphigus, pemphigoid, vasculitis syndrome, autoimmune hemolytic anemia, Goodpasture's syndrome, Behçet's disease, sarcoidosis, organ transplant rejection, etc. may be mentioned. Further, as the allergic diseases in the present invention, for example, bronchial asthma, atopic asthma, atopic dermatitis, allergic rhinitis, urticarial eruption, hayfever, drug allergy, contact dermatitis, etc. may be mentioned.

In the compound of the present invention having the formula (I): 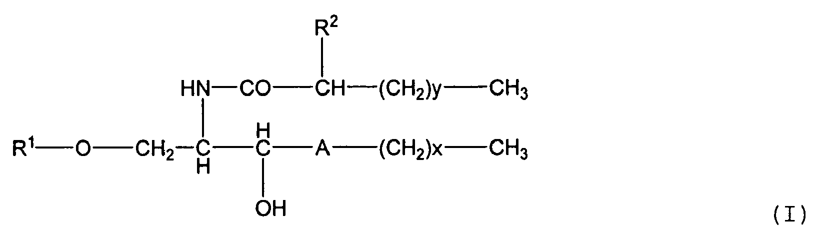 wherein R¹, R², A, x and y are as defined above, as preferable examples of the aldopyranose residue indicated by R¹, α-D-glucosyl, α-D-galactosyl, α-D-mannosyl, β-D-glucosyl, β-D-galactosyl, β-D-mannosyl, 2-deoxy-2-amino-α-D-galactosyl, 2-deoxy-2-amino-β-D-galactosyl, 2-deoxy-2-acetylamino-α-D-galactosyl, 2-deoxy-2-acetylamino-β-D-galactosyl, β-D-allopyranosyl, β-D-altropyranosyl, β-D-indosyl etc. may be mentioned, particularly preferably α substituent such as α-D-glucosyl, α-D-galactosyl, α-D-mannosyl, 2-deoxy-2-amino-α-D-galactosyl, 2-deoxy-2-acetylamino-α-D-galactosyl may be mentioned. R² indicates a hydrogen atom or hydroxy group, but is preferably a hydrogen atom. A indicates -CH₂-, -CH(OH)-CH₂- or -CH=CHCH₂-, but is preferably -CH₂- or -CH(OH)-CH₂-, particularly preferably -CH(OH)-CH₂-. x indicates an integer of 13 to 16, but is preferably an integer of 14 to 16. y indicates an integer of 0 to 25, but is preferably 15 to 25, more preferably 18 to 25, most preferably an integer of 20 to 25.

Among the compounds having the formula (I), the particularly preferable examples may be listed as follows: That is, 2-hexacosanoylamino-1-O-α-D-galactopyranosyl-D-ribo-1,3,4-docosanetriol, 2-hexacosanoylamino-1-O-α-D-galactopyranosyl-D-ribo-1,3,4-henicosanetriol, 2-hexacosanoylamino-1-O-α-D-galactopyranosyl-D-ribo-1,3,4-icosanetriol, 2-hexacosanoylamino-1-O-α-D-galactopyranosyl-D-ribo-1,3,4-nonadecanetriol, 2-pentacosanoylamino-1-O-α-D-galactopyranosyl-D-ribo-1,3,4-docosanetriol, 2-pentacosanoylamino-1-O-α-D-galactopyranosyl-D-ribo-1,3,4-henicosanetriol, 2-pentacosanoylamino-1-O-α-D-galactopyranosyl-D-ribo-1,3,4-icosanetriol, 2-pentacosanoylamino-1-O-α-D-galactopyranosyl-D-ribo-1,3,4-nonadecanetriol, 2-tetracosanoylamino-1-O-α-D-galactopyranosyl-D-ribo-1,3,4-docosanetriol, 2-tetracosanoylamino-1-O-α-D-galactopyranosyl-D-ribo-1,3,4-henicosanetriol, 2-tetracosanoylamino-1-O-α-D-galactopyranosyl-D-ribo-1,3,4-icosanetriol, 2-tetracosanoylamino-1-O-α-D-galactopyranosyl-D-ribo-1,3,4-nonadecanetriol, 2-heptacosanoylamino-1-O-α-D-galactopyranosyl-D-ribo-1,3,4-docosanetriol, 2-heptacosanoylamino-1-O-α-D-galactopyranosyl-D-ribo-1,3,4-henicosanetriol, 2-heptacosanoylamino-1-O-α-D-galactopyranosyl-D-ribo-1,3,4-icosanetriol, 2-heptacosanoylamino-1-O-α-D-galactopyranosyl-D-ribo-1,3,4-nonadecanetriol, 2-octacosanoylamino-1-O-α-D-galactopyranosyl-D-ribo-1,3,4-docosanetriol, 2-octacosanoylamino-1-O-α-D-galactopyranosyl-D-ribo-1,3,4-henicosanetriol, 2-octacosanoylamino-1-O-α-D-galactopyranosyl-D-ribo-1,3,4-icosanetriol, 2-octacosanoylamino-1-O-α-D-galactopyranosyl-D-ribo-1,3,4-nonadecanetriol, 2-nonacosanoylamino-1-O-α-D-galactopyranosyl-D-ribo-1,3,4-docosanetriol, 2-nonacosanoylamino-1-O-α-D-galactopyranosyl-D-ribo-1,3,4-henicosanetriol, 2-nonacosanoylamino-1-0-α-D-galactopyranosyl-D-ribo-1,3,4-icosanetriol, 2-nonacosanoylamino-1-O-α-D-galactopyranosyl-D-ribo-1,3,4-nonadecanetriol etc. may be mentioned.

The glycolipid having the formula (I) may be synthesized by various methods, but, for example, can be synthesized according to the methods described below: These methods will be explained successively.

First, the compound (VIa) is obtained from a known starting substance (IVa) (W02004/072091; K. Murata et al., J. Org. Chem. 2005, 70, 2398). Further, according to a known method (for example, M. Morita et al., J. Med. Chem. 1995, 38, 2176) the compounds (IVb) and (IVc) is obtained, the double bond parts of the compound (IVb) are then reduced to be converted to the compound (VIb) (step 1). From the compound (VIa), (VIb) or (IVc), the compound (VIIa), (VIIb) or (VIIc) is obtained (step 2), then selective reduction of the azide groups and an amidation reaction are used to obtain the compound (VIIIa), (VIIIb) or (VIIIc) (step 3). A glycosylation reaction of the compound (VIIa), (VIIb) or (VIIc) and compound (IX) is then used to obtain the compound (Xa), (Xb) or (Xc) (step 4). Selective reduction of the azide groups and an amidation reaction of the compound (Xa), (Xb) or (Xc) are used to obtain the compound (XIa), (XIb) or (XIc). Further, the compound (XIa), (XIb) or (XIc) can be obtained by a glycosilylation reaction of the compound (VIIIa), (VIIIb) or (VIIIc) and the compound (IX) (step 5). Finally, the protective groups of the compound (XIa), (XIb) or (XIc) can be removed to obtain the desired compound (Ia) (step 6).

### Step 1:

It is possible to synthesize the compound (VIa) from the known starting material (IVa). Further, it is possible to obtain the compounds (IVb) and (IVc) by a known method. The compound (IVb) can be converted to the compound (VIb). 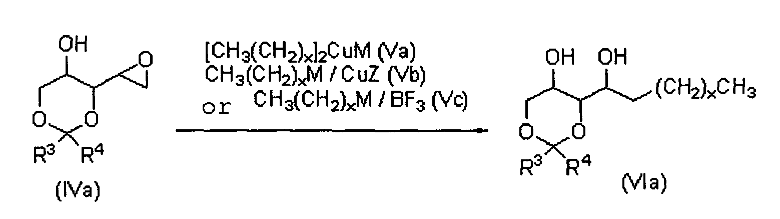 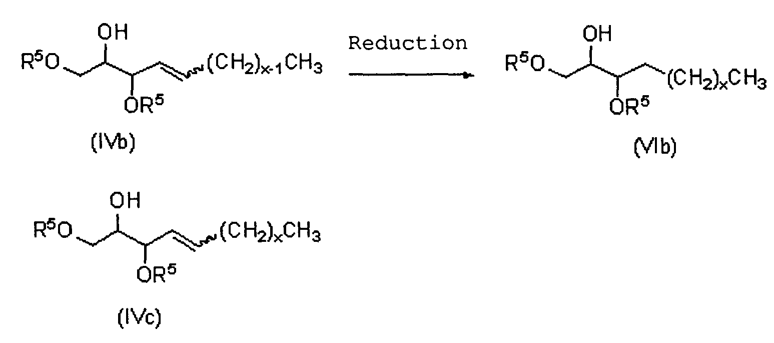 wherein x is as defined above, R³ and R⁴ may be the same or different and indicate a hydrogen atom, an alkyl group substituted or unsubstituted with a methyl group, ethyl group, isopropyl group, methoxy group, trifluoromethyl group, chlorine atom, fluorine atom, etc., an aryl group substituted or unsubstituted with a methyl group, ethyl group, isopropyl group, methoxy group, trifluoromethyl group, methoxymethyl group, chlorine atom, fluorine atom, bromine atom, iodine atom, nitro group, etc., or an aralkyl group substituted or unsubstituted with a methyl group, ethyl group, isopropyl group, methoxy group, trifluoromethyl group, methoxymethyl group, chlorine atom, fluorine atom, bromine atom, iodine atom, nitro group, etc., or R³ and R⁴ together bond to form a cyclic structure of a propylene group, butylene group or pentylene group, R⁵ indicates a benzyl group, p-methoxybenzyl group, p-nitrobenzyl group, p-methoxymethyloxybenzyl group, p-benzyloxybenzyl group, 3,4-dimethoxybenzyl group, diphenylmethyl group, or di(p-nitrophenyl)methyl group, M indicates Li, MgCl, MgBr or MgI and Z indicates a chlorine atom, bromine atom or iodine atom.

In the step from the compound (IVa) to the compound (VIa), in an inert solvent such as diethyl ether, tetrahydrofuran, dioxane, toluene, xylene, hexane, cyclohexane or a mixed solvent thereof containing copper (I) iodide, copper (I) bromide, copper (I) chloride or borofluoride, 1 to 6 equivalents of alkyllithium reagent or a Grignard reagent was added to the compound (IVa) at -78°C to 0°C, preferably -50°C to -10°C, the mixture was stirred at the same temperature, for example, for 1 to 5 hours, the compound (IVa) was added thereto and then the mixture was further stirred for 1 to 5 hours to obtain the desired compound (VIa). Further, in the step from the compound (IVb) to the compound (VIb), the compound (IVb) may be stirred in an inert solvent such as diethyl ether, dimethoxyethane, tetrahydrofuran, dioxane, toluene, xylene, ethyl acetate, chloroform, dichloromethane, methanol, water, ethanol, isopropyl alcohol or the mixed solvents thereof, optionally in the presence of a base such as sodium acetate, potassium acetate, sodium carbonate, sodium hydrogen carbonate, together with hydrazine or tosylhydrazine at 30°C to 150°C or is hydrogenated in the presence of Pd-C, Pd-CaCO₃-Pb, Pd-BaSO₄, PtO₂ etc. at a room temperature so as to convert the compound (VIb) to the compound (IVb).

The compound obtained by this reaction may be directly used as the material for the next step, but, if necessary, a purification method generally used, for example, recrystallization or column chromatography may also be utilized for purification.

### Step 2:

It is possible to convert the compound (VIa) obtained at step 1 to the compound (VIIa) or (VIIb). Further, it is possible to convert the compound (VIb) or (IVc) obtained at step 1 to the compound (VIIc). 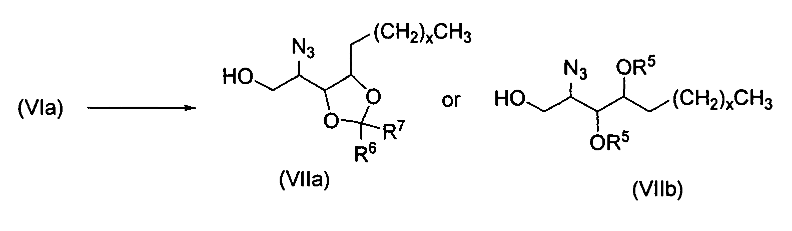 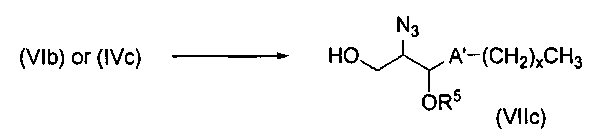 wherein x and R⁵ are as defined above, R⁵ and R⁶ may be the same or different and indicates a hydrogen atom, an alkyl group substituted or unsubstituted with a methyl group, ethyl group, isopropyl group, methoxy group, trifluoromethyl group, chlorine atom, fluorine atom, etc., an aryl group substituted or unsubstituted with a methyl group, ethyl group, isopropyl group, methoxy group, trifluoromethyl group, methoxymethyl group, chlorine atom, fluorine atom, bromine atom, iodine atom, nitro group, etc., or an aralkyl group substituted or unsubstituted with a methyl group, ethyl group, isopropyl group, methoxy group, trifluoromethyl group, methoxymethyl group, chlorine atom, fluorine atom, bromine atom, iodine atom, nitro group, etc. or R⁵ and R⁶ together bond to form a cyclic structure of a propylene group, butylene group or pentylene group and A' indicates -CH₂- or -CH=CHCH₂-.

The compound (VIa) may be reacted in an inert solvent such as methylene chloride, 1,2-dichloroethane, chloroform, carbon tetrachloride, acetonitrile, diethyl ether, tetrahydrofuran, dioxane, benzene, toluene, xylene, ethyl acetate, in the presence of a base such as triethylamine, diisopropylethylamine, pyridine, sodium carbonate, sodium hydrogen carbonate, potassium carbonate, potassium hydrogen carbonate at -20°C to 100°C, preferably -10°C to 80°C with 1 to 5 equivalents of a sulfonylation agent such as methanesulfonyl chloride, methanesulfonate anhydride, ethanesulfonyl chloride, 1-propanesulfonyl chloride, 1-butanesulfonyl chloride, trifluoromethanesulfonyl chloride, α-toluenesulfonyl chloride, benzenesulfonyl chloride, p-toluenesulfonyl chloride, p-toluenesulfonate anhydride, 4-methoxybenzenesulfonyl chloride, 4-chlorobenzenesulfonyl chloride, 2-nitrobenzenesulfonyl chloride, 3-nitrobenzenesulfonyl chloride, 4-nitrobenzenesulfonyl chloride, for example, for 1 to 72 hours and deacetalated by an ordinary method. For the deacetalation conditions, many methods described in "Protective Groups In Organic Synthesis" (John Wiley & Sons) etc. may be used. For example, the compound may be stirred in a mixture of an inorganic acid or organic acid such as hydrochloric acid, sulfuric acid, nitric acid, acetic acid, trifluoroacetic acid, methanesulfonic acid, trifluoromethanesulfonic acid and an inert solvent such as methanol, ethanol, 2-propanol, dioxane, methylene chloride, 1,2-dichloroethane, chloroform, carbon tetrachloride, benzene, toluene, xylene at -10°C to 100°C, preferably 0 to 50°C, so as to obtain the desired substance. Next, the compound this obtained is reacted in an inert solvent such as acetonitrile, diethyl ether, tetrahydrofuran, dioxane, benzene, toluene, xylene, dimethyl sulfoxide, dimethyl formamide with 1 to 50 equivalents of sodium azide and/or lithium azide at 0 to 200°C, preferably 20 to 120°C. At this time, if necessary, a base such as triethylamine, diisopropylethylamine, pyridine, sodium carbonate, sodium hydrogen carbonate, potassium carbonate, potassium hydrogen carbonate may also be added. The compound thus obtained is acetalated to obtain the compound (VIIa). As the acetal conditions, the various methods described in "Protective Groups In Organic Synthesis" (John Wiley & Sons) etc. may be used. That is, the compound may be reacted in the presence of an organic acid or inorganic acid, under solvent-less conditions, or in an inert solvent such as diethyl ether, dioxane, benzene, toluene, xylene, with an acetalation reagent at 0 to 200°C, preferably 20 to 120°C so as to obtain the desired compound (VIIa). At this time, as the acetalation reagent, acetone, 2,2-dimethoxypropane, 2-methoxypropane, 2-ethoxypropane, benzaldehyde, benzaldehyde dimethyl acetal, cyclohexanone, cyclohexanone dimethylacetal, cyclopentanone, cyclopentanone dimethylacetal, etc. may be used. Further, the compound obtained after the azidization can be tritylated on the primary hydroxyl group then have the other secondary hydroxyl groups arylmethylated, then can be detritylated so as to obtain the compound (VIIb). As the tritylation conditions, for example, the reaction conditions of 0.8 to 2 equivalents of an inert solvent such as trityl bromide or trityl chloride in diethyl ether, tetrahydrofuran, dioxane, benzene, toluene, xylene, dimethyl formamide, dimethyl sulfoxide, in the presence of a base such as lithium carbonate, potassium carbonate, sodium carbonate, sodium hydrogen carbonate, potassium hydrogen carbonate, lithium hydroxide, sodium hydroxide, potassium hydroxide, sodium hydroxide, potassium hydride, sodium, potassium, triethylamine, diisopropyl ethylamine, pyridine, lutidine at -50°C to 120°C, preferably -20°C to 80°C may be mentioned. Further, as the arylmethylation agent, benzyl chloride, benzyl bromide, p-methoxybenzyl chloride, m-methoxybenzyl chloride, p-nitrobenzyl chloride, p-nitrobenzyl bromide, etc. may be mentioned. As the arylmethylation reaction conditions, the conditions of the above tritylation may be used. Further, as the conditions for detritylation, the above various methods described in "Protective Groups In Organic Synthesis" (John Wiley & Sons) etc. may be used. For example, the reaction conditions under solvent-less conditions or in a solvent such as methylene chloride, chloroform, 1,2-dichloroethane, benzene, toluene, xylene, dioxane, water, methanol, ethanol, 2-propanol, tert-butanol in the presence of an acid such as formic acid, acetic acid, trifluoroacetic acid, hydrochloric acid, sulfuric acid, nitric acid or copper (II) sulfate at -50°C to 150°C, preferably -20°C to 100°C may be mentioned.

The compound obtained by this reaction may be directly used as the starting material for the next step, but, if necessary, a purification method generally used, for example, recrystallization or column chromatography may also be utilized for purification.

### Step 3:

The azide groups of the compound (VIIa), (VIIb) and (VIIc) obtained at step 2 may be reduced to amino groups, then converted to amide groups to obtain the compounds (VIIIa), (VIIIb) and (VIIIc). 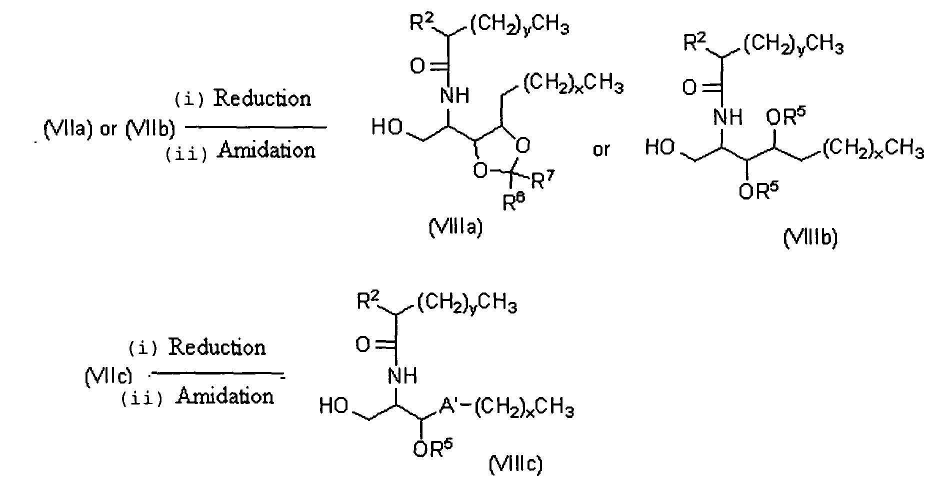 wherein R², R⁵, R⁶, R⁷, x, y and A' are as defined above.

First, the compound (VIIa), (VIIb) or (VIIc) is treated by a metal reagent such as zinc/hydrochloric acid, lithium aluminum hydride, or a triaryl phosphine or trialkyl phosphine such as triphenyl phosphine, trimethyl phosphine, triethyl phosphine, tributyl phosphine, or is hydrogenated in the presence of Pd-C, Pd-CaCO₃-Pb, Pd-BaSO₄, PtO₂, etc. at room temperature to selectively reduce the azide groups to amino groups, then an amidation reaction with carboxylic acid is used to obtain the compound (VIIIa), (VIIIb) or (VIIIc). For the amidation reaction, the various methods described in "Compendium for Organic Synthesis" (Wiley-Interscience; A Division of John Wiley & Sons) etc. may be utilized. As one example, by reacting an amine body in an inert solvent such as methylene chloride, chloroform, 1,2-dichloroethane, diethyl ether, tetrahydrofuran, dioxane, acetonitrile, benzene, toluene, xylene, dimethyl formamide in the presence of an activation agent of carboxylic acid at -50°C to 120°C, preferably -20°C to 80°C, with a corresponding carboxylic acid, the desired compound (VIIIa), (VIIIb) or (VIIIc) can be obtained. As the activation reagent of carboxylic acid, silicon tetrachloride, acetic anhydride, acetyl chloride, ethyl chlorocarbonate, 2-iodo-1-methylpyridinium iodide, 2-chloro-1-methylpyridinium iodide, diphenylphosphinyl chloride, N,N'-dicyclohexyl carbodiimide (DCC), N-hydroxybenzotriazole/DCC, 1-ethyl-3-(3-diethylaminopropyl)carbodiimide hydrochloride, ethoxyacetylene, trimethylsilylethoxyacetylene, carbodiimidazole, diphenylphosphoryl azide, diethylphosphoryl cyanidate, etc. may be mentioned. Further, if necessary, an acid such as p-toluenesulfonic acid, polyphosphoric acid, or a base such as triethylamine, diisopropylethylamine, N-methylmorpholine, pyridine, 2,6-lutidine, 4-dimethylaminopyridine may be added.

The compound obtained by this reaction may be directly used as the starting material for the next step, but if necessary, a purification method generally used, for example, recrystallization or column chromatography may also be utilized for purification.

### Step 4:

The glycosidation reaction of the compound (VIIa), (VIIb) or (VIIc) and the compound (IX) obtained at step 2 can be used to obtain the compound (Xa), (Xb) or (Xc). 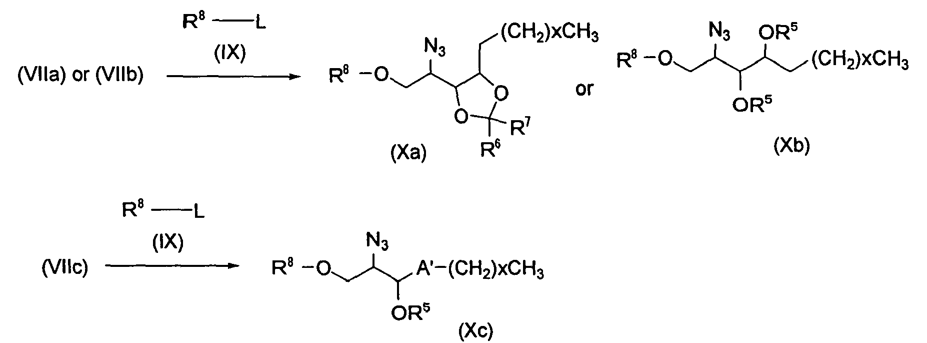 wherein R², R⁵, R⁶, R⁷ , x and A' are as defined above, R⁸ indicates an aldopyranose residue protected with a hydroxy group or amino group and L indicates a chlorine atom, bromine atom, fluorine atom or iodine atom.

The compound (VIIa), (VIIb) or (VIIc) can be reacted in an inert solvent such as hexane, cyclohexane, methylene chloride, chloroform, 1,2-dichloroethane, ether, tetrahydrofuran, acetonitrile, benzene, toluene, xylene, dioxane, dimethyl formamide, dichloromethane or a mixed solvent thereof in the presence of Lewis acid such as borotrifluoride, silver perchlorate, stannous (II) chloride, titanium tetrachloride, stannous tetrachloride, or in the presence of a halogenated ammonium salt such as tetra-n-butylammonium bromide, for example, at -100°C to 50°C, preferably -78°C to 30°C, with the compound (IX) to obtain the compound (Xa), (Xb) or (Xc). The Lewis acid or halogenated ammonium salt used in this reaction may be used alone or in combinations of several types. Further, at that time, if necessary, a molecular sieve may be added.

Further, as the aldopyranose residue protected with a hydroxy group or amino group forming R⁸, 2,3,4,6-tetra-O-benzyl-D-glucosyl, 2,3,4,6-tetra-O-benzyl-D-galactosyl, 2,3,4,6-tetra-O-benzyl-D-mannosyl, 3,4,6-tri-O-benzyl-2-deoxy-2-deoxy-2-(tert-butoxycarbonylamino)-D-galactosyl, 3,4,6-tri-O-benzyl-2-deoxy-2-acetylamino-D-galactosyl, 2,3,4,6-tetra-O-benzyl-D-allopyranosyl, 2,3,4,6-tetra-O-benzyl-β-D-altropyranosyl, 2,3,4,6-tetra-O-benzyl-β-D-indosyl, 3,4,6-tri-O-benzyl-2-deoxy-2-(dibenzylamino)-D-galactosyl, etc. may be mentioned.

The compound (Xa), (Xb) or (Xc) obtained by this reaction may be directly used as the starting material for the next step, but, if necessary, a purification method generally used, for example, recrystallization or column chromatography may also be utilized for purification.

### Step 5

By reducing the azide group of the compound (Xa), (Xb) or (Xc) obtained at step 4 to an amino group, then converting the amino group to an amide group, it is possible to obtain a compound (XIa), (XIb) or (XIc). Further, a glycosidation reaction of the compound (VIIIa), (VIIIb) or (VIIIc) obtained at step 3 and the compound (IX) can be used to obtain the compound (XIa), (XIb), or (XIc). 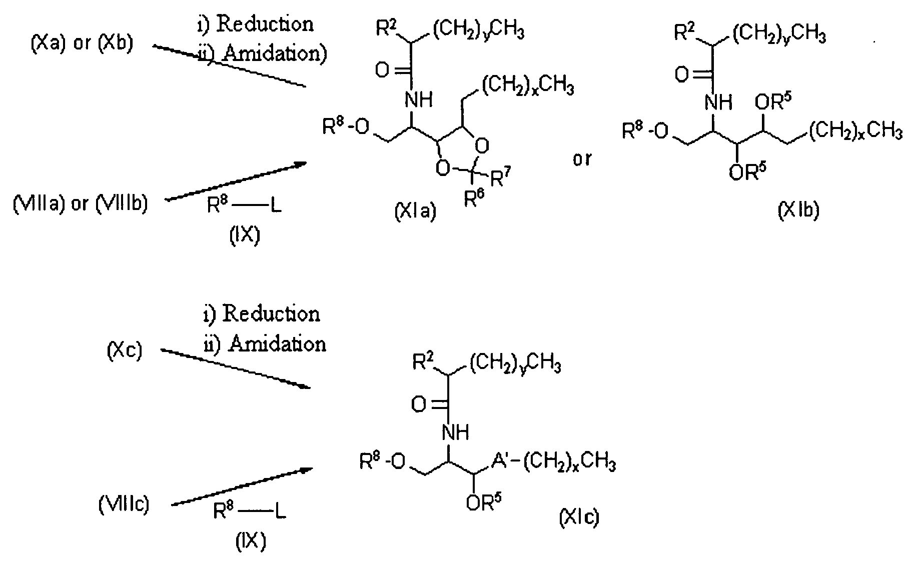 wherein R², R⁵, R⁶, R⁷, R⁸, x, y and A' are as defined above.

The compound (Xa), (Xb) or (Xc) can be converted to the compound (XIa), (XIb) or (XIc) by the same method as in step 3. Further, the compound (VIIIa), (VIIIb) or (VIIIc) can be converted to the compound (XIa), (XIb) or (XIc) by the same method as in step 4.

The compound obtained by this reaction may be directly used as the material for the next step, but, if necessary, a generally used purification method, for example, recrystallization or column chromatography may also be utilized for purification.

### Step 6:

The compound (XIa) obtained at step 5 can be deacetalated and dearylmethylated or the compound (XIb) or (XIc) can be dearylmethylated to obtain the compound (I). 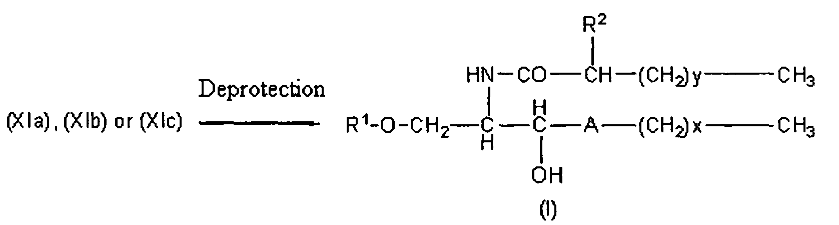 wherein R¹, R², R⁵, R⁶, R⁷, R⁸, x, y, A and A' are as defined above.

As the deprotection conditions of acetal and arylmethyl groups, the various methods described in "Protective Groups In Organic Synthesis" (John Wiley & Sons) etc. may be used. For example, as the deacetalation conditions, the method shown in step 2 may be used. Further, as the dearylmethylation conditions, the conditions of adding 4-methylcyclohexene in a solvent not participating in the reaction, such as methanol, ethanol, 2-propanol, ethyl acetate, tetrahydrofuran, dimethyl formamide in the presence of Pd-C, Pd(OH)₂, PtO₂, etc. for heating and reflux or hydrogenation at room temperature may be mentioned.

The compound obtained by this reaction may be directly used as the material for the next step, but, if necessary, a purification method generally used, for example, recrystallization or column chromatography may also be utilized for purification.

The compound having the formula (I) of the present invention may be administered alone or may be as desired prepared together with another ordinary pharmacologically acceptable known commonly used vehicle into a preparation aimed at the improvement or treatment of a chronic inflammatory disease, allergic disease, or disease, where it is known that NKT cells or stimulus of NKT cells is participating in the deterioration of disease conditions. For example, the active ingredient may be administered alone or together with a commonly used excipient as a capsule, tablet, injection or other suitable form orally or parentally. For example, capsules are prepared by mixing the powder material with lactose, starch or its derivative, a cellulose derivative, or other excipient and packing the mixture in gelatin capsules. Further, in addition to the excipient, sodium carboxymethylcellulose, alginic acid, gum arabic, or another binder and water are added and kneaded in, the mixture is granulated, if necessary, then talc, stearic acid, or another lubricant is further added and a usual tablet press is used for preparation. At the time of parental administration by injection, the active ingredient may be dissolved together with a solubility aid in sterilized distilled water or sterilized physiological saline and sealed in ampoules for preparation of injections. If necessary, a stabilizer or buffer may be included.

The dosage of the drug for improvement or treatment of autoimmune diseases, allergic diseases, or diseases in which NKT cells or stimulus to NKT cells is known to be participating in the deterioration of the disease conditions of the present invention depends on various factors such as the symptoms, age, route of administration, form of drug, number of dosages, etc. of the patient to be treated, but usually is 0.001 mg to 5000 mg/day/person, preferably 0.01 mg to 500 mg/day/person, more preferably 0.1 mg to 100 mg/day/person is suitable.

### EXAMPLES

Reference Examples and Examples will now be used to explain the present invention more specifically, but the scope of the present invention is by no means limited to these Examples.

### Reference Example 1: Synthesis of 1,3-0-benzylidene-D-arabitol (Compound 1)

D-arabitol (300 g, 1.97 mol) and benzaldehyde (261 g, 2.46 mol) were mixed, hydrogen chloride gas was bubbled into them for 50 minutes, then the mixture was stirred under an argon stream. The mixture was allowed to stand overnight, then the solid crystalline mass obtained was broken up, a 5% aqueous ammonia solution (900 ml) and toluene (600 ml) were added, and the mixture was stirred for 1 hour. The crystal obtained was obtained by filtration, then successively washed with cooled water (600 ml) and toluene (600 mlx2) and dried in vacuo to obtain the above-identified compound 295 g (yield 62.4%). mp 130-131°C; ¹H-NMR (CD₃OD) δ: 7.51-7.30 (m, 5H), 5.58 (s, 1H), 4.18 (d, 1H, J=12Hz), 4.11 (d, 1H, J=12Hz), 3.87-3.28 (m, 5H); HRMS-FAB (m/z): calcd for C₁₂H₁₇O₅ [M+H]⁺, 241.1076; found 241.1086.

### Reference Example 2: Synthesis of 1,3-O-benzylidene-5-O-toluenesulfonyl-D-arabitol (Compound 2)

To a solution of the compound 1 synthesized in Reference Example 1 (30 g, 125 mmol) dissolved in methylene chloride (240 ml), di-n-butyltin oxide (623 mg, 2.50 mmol), p-toluenesulfonyl chloride (23.8 g, 125 mmol) and triethylamine (12.9 g, 127 mmol) were added under an argon stream. The mixture was stirred at room temperature for 20 hours, then the organic layer was washed with water (100 mlx3) and dried over sodium sulfate. The solvent was concentrated and the residue obtained was purified by column chromatography (methylene chloride:methanol=40:1) to obtain a crude crystal. Finally, the crude crystal was recrystallized (n-hexane:ethyl acetate=1:1) to obtain the above-identified compound 39.7 g (yield 80.8%).

¹H-NMR (DMSO-d₆) δ: 7.73 (d, 2H, J=8.2Hz), 7.37-7.24 (m, 7H), 5.43 (s, 1H), 5.34 (d, 1H, J=6.1Hz), 4.77 (d, 1H, J=6.5Hz), 4.11 (dd, 1H, J=9.8, 1.9Hz), 4.04-3.85 (m, 4H), 3.71 (d, 1H, J=9.2Hz), 3.59 (d, 1H, J=5.7Hz), 2.32 (s, 3H);
¹³C-NMR (CDCl₃) δ: 145.1, 137.3, 132.5, 129.9, 129.1, 128.2, 128.0, 125.8, 101.0, 77.9, 72.4, 70.9, 67.6, 62.7, 21.6; MS-ESI (m/z): 395 [M+H]⁺; HRMS-FAB (m/z): calcd for C₁₉H₂₃O₇S [M+H]⁺, 395.1164; found 395.1189.

### Reference Example 3: Synthesis of 4,5-anhydro-1,3-O-benzylidene-D-arabitol (Compound 3)

A solution of the compound 2 synthesized in Reference Example 2 (30 g, 76 mmol) dissolved in anhydrous tetrahydrofuran (191 ml) was ice cooled under an argon stream. Into this, while holding a temperature of 3 to 5°C, potassium-t-butoxide (9.11 g, 81.2 mmol) was added over 20 minutes. At the same temperature, the mixture was stirred for 1.5 hours, then water (76 ml) was added, while ice cooling, and the mixture was neutralized with 1M hydrochloric acid (pH=7.5) and the product was extracted over methylene chloride (76 mlx3). The organic layer was dried over sodium sulfate, then the solvent was concentrated. The residue obtained was purified by column chromatography (methylene chloride:methanol=50:1) to obtain a crude crystal. Finally, n-hexane (90 ml) was added to the crude crystal and the resultant mixture stirred at room temperature for 30 minutes to obtain the above-identified compound 15.9 g (yield 94%).

¹H-NMR (CDCl₃) δ: 7.52-7.34 (m, 5H), 5.57 (s, 1H), 4.25 (dd, 1H, J=12, 1.8Hz), 4.08 (dd, 1H, J=12, 1.2Hz), 3.78-3.75 (m, 2H), 3.35-3.31 (m, 1H), 2.93-2.84 (m, 3H); ¹³C-NMR (CDCl₃) δ: 137.4, 129.3, 128.4, 126.0, 101.4, 79.7, 72.3, 64.4, 50.8, 45.9; MS-ESI (m/z): 223 [M+H]⁺; HRMS-FAB (m/z): calcd for C₁₂H₁₅O₄ [M+H]⁺, 223.0971; found 223.0895.

### Example 1: Synthesis of 1,3-O-benzylidene-D-arabino-1,2,3,4-docosanetetraol (Compound 4)

To a suspension of copper iodide (I) (3.2 g, 16.8 mmol) in anhydrous tetrahydrofuran (210 ml), 1.58M magnesium n-heptadecyl bromide (67 mmol in tetrahydrofuran solution) was dropwise added at -40°C. The mixture was stirred at -15°C for 30 minutes. Next, a solution of the compound 3 synthesized in Reference Example 3 (5 g, 22.5 mmol) dissolved in anhydrous tetrahydrofuran (100 ml) was dropwise added at -20°C, the mixture was stirred at the same temperature for 4.5 hours, then the mixture was stirred at room temperature overnight. To the reaction mixture, a saturated aqueous ammonium chloride solution was added, the product was extracted by ethyl acetate, then the organic layer was washed with saturated saline, dried over sodium sulfate, filtered, then concentrated in vacuo. The residue obtained was purified by silica gel column chromatography (chloroform:ethyl acetate =2:1 to 1:2) to obtain the above-identified compound 3.75g (yield 36%). White powder;
¹H-NMR (CDCl₃)δ: 7.53 (d, 2H), 7.39 (m, 3H), 5.60 (s, 1H), 4.28 (dd, 1H), 4.05 (d, 1H), 4.00-3.80 (m, 2H), 3.27 (d, 1H), 2.39 (d, 1H), 1.85-1.20 (m, 34H),0.89 (t, 3H).

### Example 2: Synthesis of 1,3-O-benzylidene-2-O-methanesulfonyl-D-arabino-1,2,3,4-docosanetetraol (Compound 5)

To a solution of the compound 4 synthesized in Example 1 (1 g, 2.2 mmol) in anhydrous pyridine (30 ml) and anhydrous tetrahydrofuran (20 ml), methanesulfonyl chloride (0.38 g, 3.3 mmol) was gradually dropwise added at -5°C. The mixture was stirred at that temperature for 18 hours, then excess methane sulfonyl chloride was quenched by an addition of 5 ml of methanol, then was stirred at room temperature overnight. The reaction mixture was concentrated in vacuo, then heptane was used to cause azeotropic removal of excess salvents (2 times), then the residue obtained was dissolved in chloroform (500 ml) and was washed with water (2 times). The organic layer was separated, dried over sodium sulfate, filtered, then concentrated in vacuo. The residue obtained was purified by silica gel column chromatography (chloroform:ethyl acetate =10:1) to obtain the above-identified compound 780 mg (yield 66.7%). White powder;
¹H-NMR (CDCl₃)δ: 7.49 (d, 2H), 7.37 (m, 3H), 5.59 (s, 1H), 4.99 (m, 1H), 4.52 (dd, 1H), 4.16 (d, 1H), 3.90-3.70 (m, 2H), 3.19 (s, 3H), 2.78 (d, 1H),1.90-1.20 (m, 34H), 0.88 (t, 3H).

### Example 3: Synthesis of 2-O-methanesulfonyl-D-ribo-1,3,4-docosanetriol (Compound 6)

To a solution of the compound 2 synthesized in Example 2 (10 g, 18.5 mmol) dissolved in methanol (110 ml), chloroform (180 ml) and anhydrous tetrahydrofuran (150 ml), 20%Pd (OH)₂/C (1.7 g) was added. The mixture was hydrogenated under atmospheric pressure at 45°C. After the completition of the reaction, chloroform (200 ml) was added, the catalyst was removed by filtration, and the filtrate was concentrated in vacuo to obtain the above-identified compound 8.27 g (yield 98.8%). White powder;
¹H-NMR (DMSO-d6) δ: 4.73 (t, 1H), 3.64 (m, 2H), 3.38 (m, 2H), 3.16 (s, 3H), 1.75-1.15 (m, 34H), 0.86 (t, 3H).

### Example 4: Synthesis of 2-azido-D-ribo-1,2,3,4-docosanetriol (Compound 7)

To a solution of the compound 6 synthesized in Example 3 (8.26 g, 18.24 mmol) dissolved in anhydrous dimethyl formamide (150 ml), sodium azide (2.37 g, 36.41 mmol) was added. The mixture was stirred at 95°C for 5 hours, then stirred at room temperature overnight. To the reaction mixture, water (1L) was added, then the product was extracted with ethyl acetate (2L). The extract was dried over sodium sulfate, filtered, then concentrated in vacuo. The crude crystal obtained was purified by silica gel column chromatography (n-hexane:ethyl acetate=3:2) to obtain the above-identified compound 5.33 g (yield 70%). Light brown colored solid;

¹H-NMR (DMSO-d6) δ: 4.99 (d, 1H), 4.87 (t, 1H), 4.51 (m, 1H), 3.80-3.70 (m, 1H), 3.65-3.45 (m, 2H), 1.65-1.15 (m, 34H), 0.85 (t, 3H).

### Example 5: Synthesis of 2-azido-3,4-O-isopropylidene-D-ribo-1,3,4-docosanetriol (Compound 8)

To a solution of the compound 7 synthesized in Example 4 (7.19 g, 18 mmol) dissolved in acetone (150 ml), concentrated sulfuric acid (0.1 g) was added. The mixture was stirred at room temperature for 3 hours. After the completition of the reaction, triethylamine (3.64 g, 36 mmol) was added, the mixture was stirred at room temperature for 1 hour, then water (800 ml) was added to the reaction mixture and the product was extracted with ethyl acetate (800 ml) and toluene (400 ml). The extract was dried over sodium sulfate, filtered, then concentrated in vacuo. The crude crystal obtained was purified by silica gel column chromatography (n-hexane:ethyl acetate=8:1 to 6:1) to obtain the above-identified compound 44.04g (yield 50.5%). White powder;
¹H-NMR (CDCl₃)δ: 4.17 (m, 1H), 4.05-3.91 (m, 2H), 3.86 (m, 1H), 346 (m, 1H), 2.11 (m, 1H), 1.65-1.15 (m, 34H), 1.43 (s, 3H), 1.34 (s, 3H), 0.88 (t, 3H).

### Example 6: Synthesis of 2-azido-l-O-(2,3,4,6-tetra-O-benzyl-α-D-galactopyranosyl)-3,4-O-isopropylidene-D-ribo-1,3,4-docosanetriol (Compound 9)

To dried molecular sieve (4A, powder) (250 mg), a solution of the compound 8 synthesized in Example 5 (101 mg, 0.23 mmol) and 2,3,4,6-tetra-O-benzyl-α-D-galactopyranosyl bromide (280 mg, 0.46 mmol) dissolved in toluene (4.5 ml) and dimethyl formamide (4.5 ml) was added. Next, tetra-n-butyl ammonium bromide (218 mg, 0.68 mmol) was added and the mixture was stirred for 4 days. To the reaction mixture, ethyl acetate (200 ml) was added. The mixture was washed with saturated aqueous sodium hydrogen carbonate solution and water. The organic layer was dried over sodium sulfate, filtered, then concentrated in vacuo. The residue obtained was purified by silica gel column chromatography (n-hexane:ethyl acetate=25:1 to 8:1) to obtain the above-identified compound 107 mg (yield 48%).
¹H-NMR (CDCl₃)δ: 7.39-7.23 (m, 20H), 4.96-4.92 (m, 2H), 4.85 (d, 1H, J=12Hz), 4.80 (d, 1H, J=12Hz), 4.73-4.68 (m, 2H), 4.57 (d, 1H, J=12Hz), 4.48 (d, 1H, J=12Hz), 4.40 (d, 1H, J=12Hz), 4.12-3.91 (m, 7H), 3.72 (dd, 1H, J=10.8, 6.6Hz), 3.54-3.44 (m, 3H), 1.59-1.20 (m, 40H), 0.88 (t, 3H, J=6.8Hz); MS-ESI (m/z): 985 (M+Na).

### Example 7: Synthesis of 2-amino-1-O-(2,3,4,6-tetra-O-benzyl-α-D-galactopyranosyl)-3,4-O-isopropylidene-D-ribo-1,3,4-docosanetriol (Compound 10)

To a solution of the compound 9 synthesized in Example 6 (87 mg, 0.09 mmol) dissolved in ethanol (9 ml) and methylene chloride (3 ml), palladium-calcium carbonate (lead detoxified) (Lindlar's catalyst) (280 mg) was added. The mixture was stirred at atmospheric pressure and room temperature overnight for hydrogenation. The catalyst was removed by filtration and the filtrate was concentrated in vacuo to obtain the above-identified compound 80 mg (yield 94%).
¹H-NMR (CDCl₃)δ: 7.39-7.13 (m, 20H), 4.95-4.92 (m, 2H), 4.83-4.78 (m, 2H), 4.74 (d, 1H, J=12Hz), 4.68 (d, 1H, J=12Hz), 4.47 (d, 1H, J=12Hz), 4.40 (d, 1H, J=12Hz), 4.11-4.04 (m, 2H), 3.99-3.91 (m, 4H), 3.87 (dd, 1H, J=9.0, 5.5Hz), 3.55-3.52 (m, 2H), 3.39 (dd, 1H, J=10.2, 7.6Hz), 3.07-3.02 (m, 1H), 1.53-1.20 (m, 40H), 0.88 (t, 3H, J=6.8Hz); MS-ESI (m/z): 937 (M+H).

### Example 8: Synthesis of 2-hexacosanoylamino-1-O-(2,3,4,6-tetra-O-benzyl-α-D-galactopyranosyl)-3,4-O-isopropylidene-D-ribo-1,3,4-docosanetriol (Compound 11)

To a mixed suspension of the compound 10 synthesized in Example 7 (560 mg, 0.6 mmol), n-hexacosanic acid (270 mg, 0.69 mmol) and 1-hydroxyazabenzotriazole (12 mg, 0.087 mmol) in dimethyl formamide (45 ml) and methylene chloride (25 ml), triethylamine (0.2 ml, 1.4 mmol) and 1-ethyl-3-(3-dimethylaminopropyl)carbodiimidic hydrochloride (210 mg, 1.1 mmol) were added under ice cooling, then the mixture was stirred at room temperature for 5 days. The reaction mixture was diluted with ethyl acetate (1.5L), then washed with saturated sodium hydrogen carbonate aqueous solution and water. The organic layer was dried over sodium sulfate, filtered and concentrated in vacuo. The residue obtained was purified by silica gel column chromatography (n-hexane:ethyl acetate=4:1) to obtain the above-identified compound 340 mg (yield 43%). White powder;

¹H-NMR (CDCl₃)δ: 7.41-7.23 (m, 20H), 6.27 (d, 1H, J=8.7Hz), 4.92 (d, 1H, J=12Hz), 4.90 (d, 1H, J=3.7Hz), 4.83-4.78 (m, 2H), 4.75 (d, 1H, J=12Hz), 4.66 (d, 1H, J=11Hz), 4.58 (d, 1H, J=12Hz), 4.49 (d, 1H, J=12Hz), 4.37 (d, 1H, J=12Hz), 4.12-4.02 (m, 4H), 3.98 (t, 1H, J=6.2Hz), 3.93-3.88 (m, 3H), 3.62-3.52 (m, 2H), 3.36 (dd, 1H, J=9.4, 5.5Hz), 2.08-1.96 (m, 2H), 1.54-1.39 (m, 7H), 1.31-1.22 (m, 79H), 0.90-0.86 (m, 6H); MS-ESI (m/z): 1338 (M+Na).

### Example 9: Synthesis of 2-hexacosanoylamino-1-O-(2,3,4,6-tetra-O-benzyl-α-D-galactopyranosyl)-D-ribo-1,3,4-docosanetriol (Compound 12)

A solution of the compound 11 synthesized in Example 8 (53 mg, 0.04 mmol) dissolved in methanol (1.5 ml)/methylene chloride (6 ml)/4N hydrochloric acid-dioxane (120 µl) was stirred at room temperature for 2 hours, then concentrated in vacuo. The residue obtained was purified by silica gel column chromatography (hexane:ethyl acetate=2:1) to obtain the above-identified compound 35 mg (yield 68%). White powder;
¹H-NMR (CDCl₃)^{™}: 7.40-7.25 (m, 20H), 6.38 (d, 1H, J=8.4Hz), 4.93-4.87 (m, 2H), 4.84 (d, 1H, J=3.8Hz), 4.79-4.73 (m, 2H), 4.67 (d, 1H, J=12Hz), 4.56 (d, 1H, J=11Hz), 4.47 (d, 1H, J=12Hz), 4.38 (d, 1H, J=12Hz), 4.21-4.18 (m, 1H), 4.04 (dd, 1H, J=10, 3.8Hz), 3.97 (d, 1H, J=1.9Hz), 3.89-3.84 (m, 4H), 3.81 (d, 1H, J=8.3Hz), 3.51-3.43 (m, 4H), 2.13-2.10 (m, 3H), 1.62-1.54 (m, 4H), 1.46-1.25 (m, 76H), 0.90-0.86 (m, 6H); MS-ESI (m/z): 1275 (M+H).

### Example 10: Synthesis of 2-hexacosanoylamino-1-O-α-D-galactopyranosyl-D-ribo-1,3,4-docosanetriol (Compound 13)

To a solution of the compound 12 synthesized in Example 9 (325 mg, 0.255 mmol) dissolved in methanol (70 ml)/methylene chloride (40 ml), palladium hydroxide (150 mg) was added. The mixture was stirred at atmospheric pressure and room temperature for 4 hours for hydrogenation. The catalyst was removed by filtration and the filtrate was concentrated in vacuo to obtain the above-identified compound 206 mg (yield 88%). White powder (recrystallized by ethanol): Rf value (TLC)=0.22 (chloroform:methanol=7:1);
¹H-NMR (py-d5) δ: 8.50 (d, 1H, J=8.8Hz), 6.98 (brs, 1H), 6.64 (brs, 1H), 6.56 (brs, 1H), 6.46 (brs, 1H), 6.34 (brs, 1H), 6.10 (brs, 1H), 5.60 (d, 1H, J=3.8Hz), 5.32-5.25 (m, 1H), 4.71-4.67 (m, 2H), 4.57-4.51 (m, 2H), 4.44-4.33 (m, 6H), 2.45 (t, 2H, J=7.4Hz), 2.35-2.25 (m, 1H), 2.00-1.62 (m, 5H), 1.50-1.18 (m, 74H), 0.90-0.85 (m, 6H); MS-ESI (m/z): 915 (M+H); MS-MALDI (m/z):[M+Na]⁺ calcd for C₅₄H₁₀₇NO₉·Na [M+Na]⁺, 937.418; found 937.09.

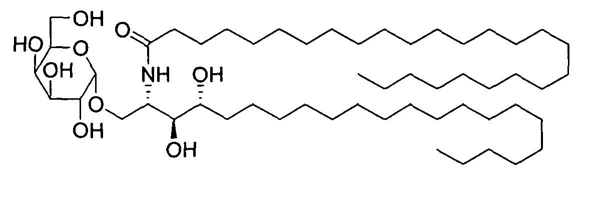

### Suppressive Effect of Synthesized Glycolipid in K/Bxn Serum Transferred Arthritis

(A) C57BL/6J mice (8 weeks old, female) were intraperitoneally administered K/BxN serum in amounts of 150 µl to induce arthritis. The arthritis score was judged by observation in the following way.
   The arthritis was scored as follows:
   0: no symptoms,
   1: swelling or reddening of one digit,
   2: swelling or reddening of two or more digits or a relatively large joint such as a wrist or ankle,
   3: swelling and reddening of upper or lower limbs,
   4: maximal swelling of upper or lower limbs.

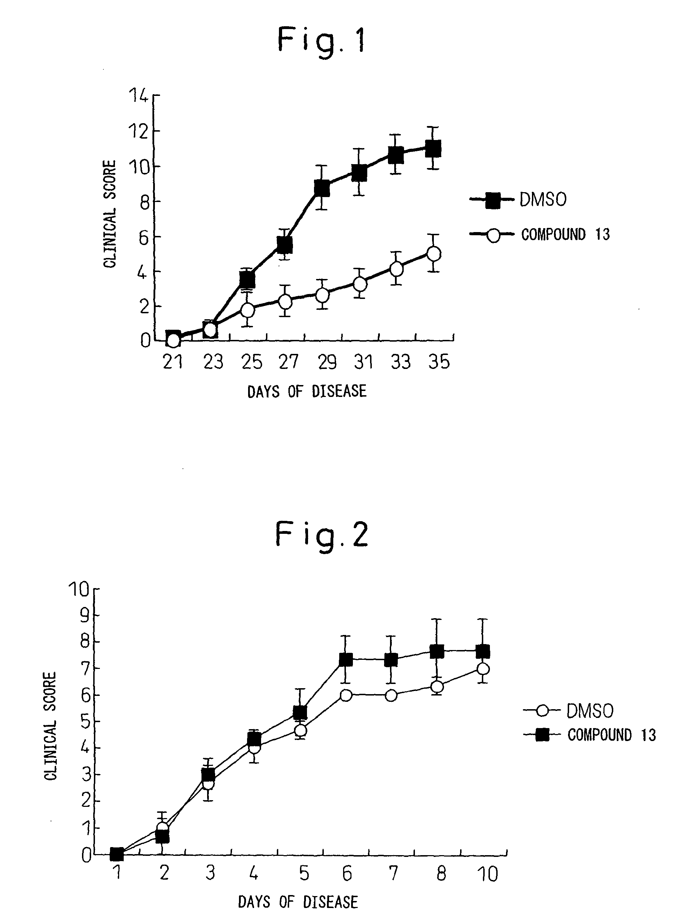

   The total of the two upper limbs and two lower limbs was used as the score.
   The compound was dissolved in 10% DMSO/PBS and was intraperitoneally administered 2 µg/mouse twice a week from the day of administration of the serum. For the control group, only 10% DMSO/PBS was administered. The administration of the compound remarkably suppressed the arthritis score (see FIG. 1).
(B) The suppressive effect of the compound was not observed in NKT cells deficient Jα18 knockout mice. These results indicated that NKT cells are required for the suppressive effect on arthritis of the compound (see Fig. 2).

### Suppressive Effect of Synthesized Glycolipid in Collagen Induced Arthritis

DBA1 mice (8 weeks old, male) were subcutaneously immunized with bovine type II collagen emulsified with Freund's Complete Adjuvant, then 3 weeks later subcutaneously immunized with bovine type II collagen along with Freund's Incomplete Adjuvant to induce arthritis. The arthritis score was evaluated by observation in the following way.

The arthritis was scored as follows:
0: no symptoms,
1: swelling or reddening of one digit,
2: swelling or reddening of two or more digits or a relatively large joint such as a wrist or ankle,
3: swelling and reddening of upper or lower limbs,
4: maximal swelling of upper or lower limbs.

The total of the two upper limbs and two lower limbs was used as the score.

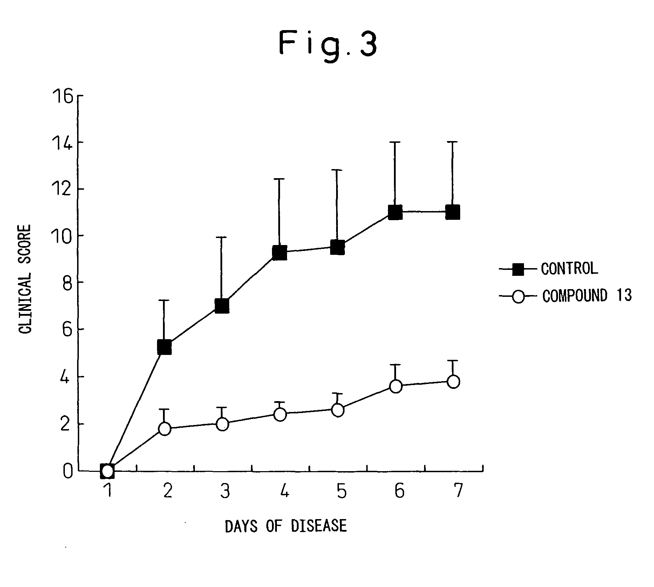

The compound was dissolved in 10% DMSO/PBS and was intraperitoneally administered 2 µg/mouse twice a week from the day of administration of the serum. For the control group, only 10% DMSO/PBS was administered. The administration of the compound remarkably suppressed the arthritis score (see FIG. 3).

### Effect of the Compound on NKT Cells

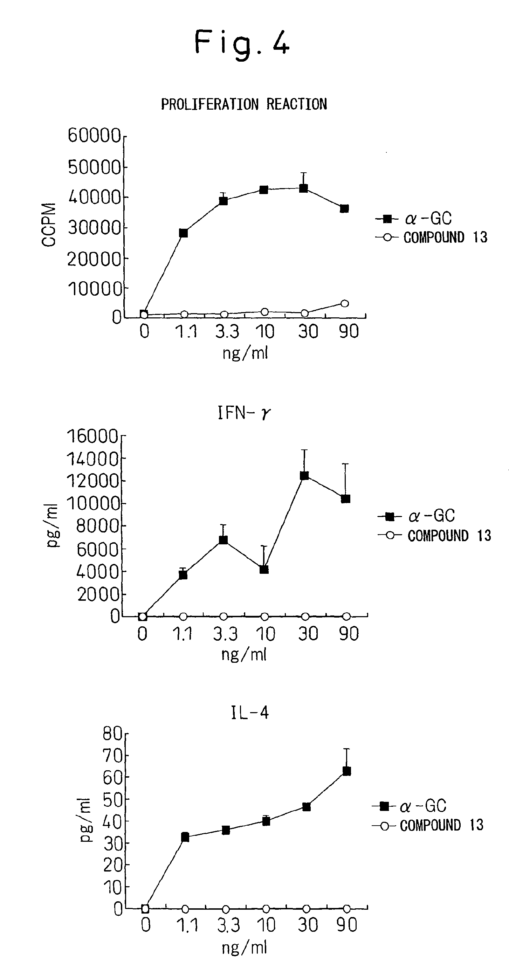

Mononuclear cells were isolated from the liver of C57BL/6J mice (8 weeks old, female) and are incubated with the compound for 48 hours. The cytokine in the supernatant was measured by the ELISA method, and the cell proliferative response was measured by the uptake of tritium thymidine. α-GC induced cell proliferation, IFN-γ production, and IL-4 production, but the compound did not react in any way (see FIG. 4).

### Effect of Preadministration of the Compound on NKT Cells

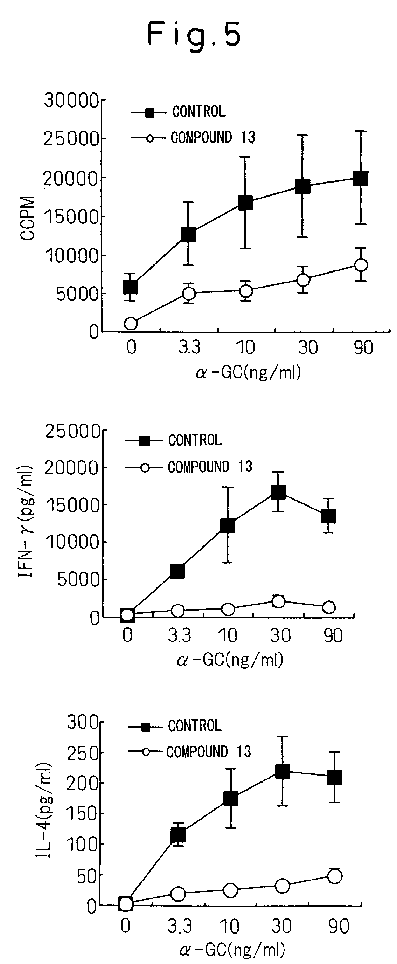

C57BL/6J mice (8 weeks old, female) were intraperitoneally administered with K/BxN serum in an amount of 150 µl to induce arthritis. The compound was administered three times every two days in an amount of 2 µg/mouse. For the control group, only 10% DMSO/PBS was administered. Two days after the final administration, mononuclear cells were isolated from the liver and incubated together with α-GC for 48 hours, the cytokine in the supernatant was measured by the ELISA method, and the cell proliferative response was measured by uptake of tritium thymidine. In the group preadministered with the compound, cell proliferation, IFN-γ production, and IL-4 production by the treatment of α-GC could not be observed. These results clearly indicated that the present invention compound suppressed the antigen stimulation of NKT cells (see FIG. 5).

### Suppression of Cellular Infiltration in Alveolus Washings in Bronchial Asthma Model (C57BL6J Mice)

C57BL/6J mice (8 weeks old, female) were intraperitoneally administered with ovalubumin (OVA) in amounts of 50 µg/mouse after blending with alum in an amount of 2.25 mg/mouse on Day 0 and Day 7.

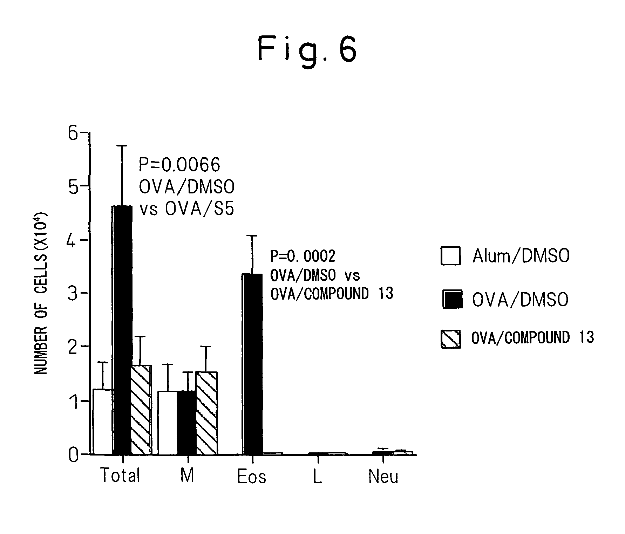

Starting from day 18, the mice were made to inhale OVA for three consecutive days at a concentration of 10 mg/ml. The compound was intraperitoneally administered before inhalation in an amount of 2 µg/mouse. One day after the final inhalation, the alveoli were washed and the cell compositions were studied. The cellular infiltration in the compound-treated group was remarkably suppressed compared with the control group (OVA/DMSO). Further, the eosinophils infiltration characteristic of asthma was remarkably suppressed (see FIG. 6).

### Suppression of Cytokine in Alveolus Washings In Bronchial Asthma Model (C57BL6J Mice)

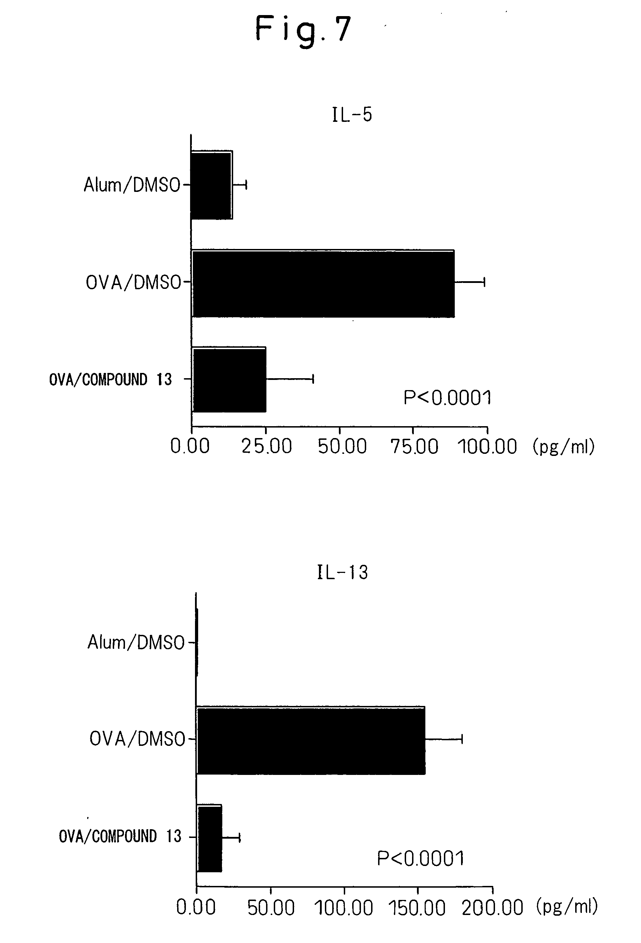

C57BL/6J mice (8 weeks old, female) were intraperitoneally administered with OVA in amounts of 50 µg/mouse after blending with alum in an amount of 2.25 mg/mouse on Day 0 and Day 7. Starting from day 18, the mice were made to inhale OVA for three consecutive days at a concentration of 10 mg/ml. The compound was intraperitoneally administered before inhalation in an amount of 2 µg/mouse. One day after the final inhalation, the alveoli were washed and the cytokine in the alveolus washings was measured using the ELISA method. Compared with the control group (OVA/DMSO), both of IL-5 and IL-13 were remarkably suppressed in the compound-treated group (see FIG. 7).

### Pathological Findings of Lungs in Bronchial Asthma Model (C57BL6J Mice)

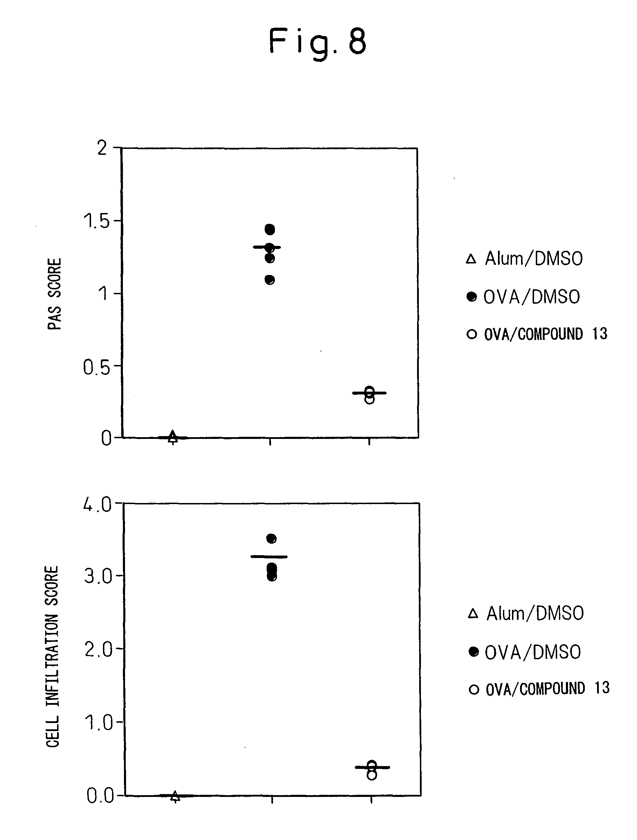

C57BL/6 mice (8 weeks, female) were intraperitoneally administered with OVA in amounts of 40 µg/mouse after blending with alum in amounts of 2.25 mg/mouse on Day 0 and Day 7. Starting from day 18, the mice were made to inhale OVA at a concentration of 10 mg/ml. The compound was intraperitoneally administered before inhalation in an amount of 2 µg/mouse. On the day after the final inhalation day, the lung tissue was excised and pathologically analyzed. Compared with the control group (OVA/DMSO), cell infiltration and the proliferation of PAS positive goblet cells were preferentially suppressed in the compound-treated group (see FIG. 8). The histological score was judged as follows: That is, the cell infiltration is scored as
0: normal,
1: small amount of cell infiltration,
2: one layer of cell infiltration,
3: two to four layers of cell infiltration, and
4: more than four layers of cell infiltration.
Further, the PAS was evaluated score as 0: goblet cells less than 5% of aveoli circumference,
1: goblet cells 5% to 25% of aveoli circumference,
2: goblet cells 25% to 50% of aveoli circumference,
3: goblet cells 50% to 75% of aveoli circumference,
4: goblet cells more than 75% of aveoli circumference.

### Suppression of Cytokine in Alveoli Washings in Bronchial Asthma Model

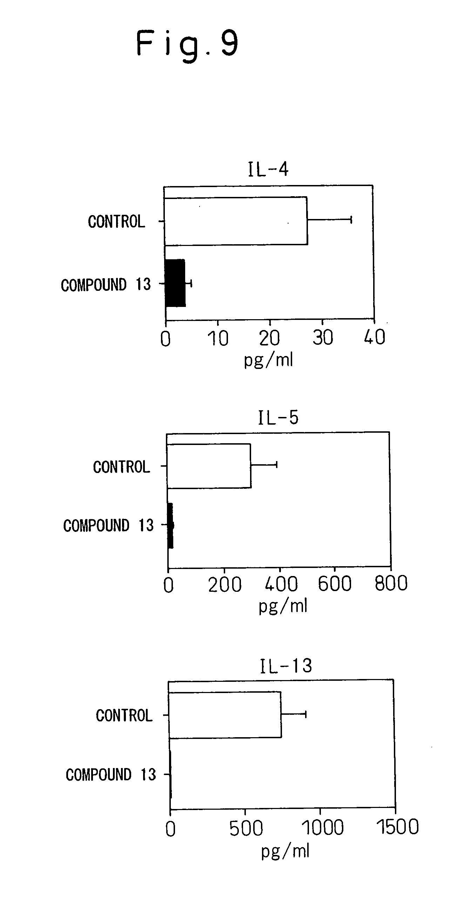

Balb/c mice (8 weeks old, female) were intraperitoneally administered with OVA in amounts of 20 µg/mouse after blending with alum in an amount of 2.25 mg/mouse on Day 0 and Day 7. Starting from day 18, the mice were made to inhale OVA for three consecutive days at a concentration of 5 mg/ml. The compound was intraperitoneally administered before inhalation in an amount of 2 µg/mouse. One day after the final inhalation, the alveoli were washed and the cytokine in the alveolus washings was measured using the ELISA method. Compared with the control group (OVA/DMSO), both of IL-5 and IL-13 were remarkably suppressed in the compound group (see FIG. 9).

### INDUSTRIAL APPLICABILITY

The glycolipid derivative SGL having the above formula (I) according to the present invention suppresses auto-antibody induced inflammation reactions and is useful as a therapeutic drug for autoimmune arthritis and other chronic inflammatory diseases, bronchial asthma and other allergic diseases, and other diseases in which NKT cells are involved in deterioration of the disease conditions.

## Claims

1. A therapeutic drug for diseases, in which NKT cells or stimulus of NKT cells is involved in deterioration of disease conditions, comprising, as an active ingredient, a glycolipid derivative having the formula (I): 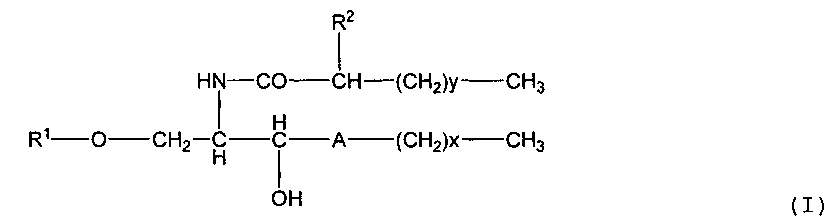 wherein R¹ indicates an aldopyranose residue, R² indicates a hydrogen atom or a hydroxy group, A indicates -CH₂-, -CH(OH)-CH₂- or -CH=CHCH₂-, x indicates an integer of 13 to 16 and y indicates an integer of 0 to 25 or its pharmacologically acceptable hydrate or solvate.

2. A therapeutic drug for an allergic disease comprising, as an active ingredient, a glycolipid derivative or its pharmacologically acceptable hydrate or solvate according to in claim 1.

3. A therapeutic drug for a chronic inflammatory disease comprising, as an active ingredient, a glycolipid derivative or its pharmacologically acceptable hydrate or solvate according to claim 1.

4. A therapeutic drug for a disease, in which NKT cells or stimulus of NKT cells is involved in deterioration of disease conditions, comprising, as an active ingredient, a glycolipid derivative or its pharmacologically acceptable hydrate or solvate according to claim 1 wherein R¹ in the formula (I) indicates α-D-galactopyranosyl.

5. A therapeutic drug for an allergic disease comprising, as an active ingredient, a glycolipid derivative according to claim 1 wherein R¹ in the formula (I) indicates α-D-galactopyranosyl or its pharmacologically acceptable hydrate or solvate.

6. A therapeutic drug for a chronic inflammatory disease, containing as an active ingredient a glycolipid derivative or its pharmacologically acceptable hydrate or solvate according to claim 1 wherein R¹ in the formula (I) indicates α-D-galactopyranosyl.

7. A therapeutic drug for diseases, in which NKT cells or stimulus of NKT cells is involved in deterioration of disease conditions, comprising, as an active ingredient, a glycolipid derivative or its pharmacologically acceptable hydrate or solvate according to claim 1 wherein, in the formula (I), R¹ indicates α-D-galactopyranosyl and A indicates -CH₂- or -CH(OH)-CH₂-.

8. A therapeutic drug for an allergic disease comprising, as an active ingredient, a glycolipid derivative or its pharmacologically acceptable hydrate or solvate according to claim 1 wherein, in the formula (I), R¹ indicates α-D-galactopyranosyl and A indicates -CH₂- or -CH (OH) -CH₂- .

9. A therapeutic drug for a chronic inflammatory disease comprising, as an active ingredient, a glycolipid derivative or its pharmacologically acceptable hydrate or solvate according to claim 1, wherein, in the formula (I), R¹ indicates α-D-galactopyranosyl and A indicates -CH₂- or -CH(OH)-CH₂-.

10. A therapeutic drug for diseases, in which NKT cells or stimulus of NKT cells is involved in deterioration of disease conditions, comprising, as an active ingredient, a glycolipid derivative or its pharmacologically acceptable hydrate or solvate according to claim 1, wherein in the formula (I), R¹ indicates α-D-galactopyranosyl, R² indicates a hydrogen atom and A indicates -CH₂- or -CH(OH)-CH₂-.

11. A therapeutic drug for an allergic disease, comprising, as an active ingredient, a glycolipid derivative or its pharmacologically acceptable hydrate or solvate according to claim 1, wherein, in the formula (I), R¹ indicates α-D-galactopyranosyl, R² indicates a hydrogen atom and A indicates -CH₂- or -CH(OH)-CH₂-.

12. A therapeutic drug for a chronic inflammatory disease comprising, as an active ingredient, a glycolipid derivative or its pharmacologically acceptable hydrate or solvate according to claim 1, wherein, in the formula (I), R¹ indicates α-D-galactopyranosyl, R² indicates a hydrogen atom and A indicates -CH₂- or -CH(OH)-CH₂-.

13. A glycolipid derivative having the formula (I'): 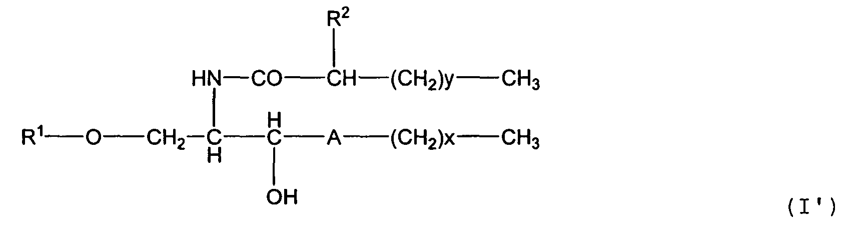 wherein R¹ indicates α-D-galactopyranosyl, R² indicates a hydrogen atom, A indicates -CH(OH)-CH₂-, x indicates an integer of 13 to 16 and y indicates an integer of 0 to 25 and its pharmacologically acceptable hydrates or solvates.

14. A glycolipid derivative and its pharmacologically acceptable hydrates or solvates as claimed in claim 13, wherein said glycolipid derivative is 2-hexacosanoylamino-1-O-α-D-galactopyranosyl-D-ribo-1,3,4-docosanetriol, 2-hexacosanoylamino-1-O-α-D-galactopyranosyl-D-ribo-1,3,4-henicosanetriol, 2-hexacosanoylamino-1-O-α-D-galactopyranosyl-D-ribo-1,3,4-icosanetriol, or 2-hexacosanoylamino-1-O-α-D-galactopyranosyl-D-ribo-1,3,4-nonadecanetriol.
